# EUROPÄISCHE PATENTSCHRIFT

(11) **EP 0 694 294 B1**
(45) Veröffentlichungstag und Bekanntmachung des Hinweises auf die Patenterteilung: **05.04.2000**
(21) Anmeldenummer: 95110318.3
(22) Anmeldetag: 03.07.1995
(51) Int. Cl.: A61F 2/34, A61F 2/46

(54) **Pfannenteil einer Gelenkprothese**
Socket of a joint prosthesis
Cupule d'une prothèse d'articulation

(30) Priorität: 28.07.1994 CH 238794
(43) Veröffentlichungstag der Anmeldung: 31.01.1996
(73) Patentinhaber: Hermann, Werner, CH-6312 Steinhausen (CH)
(72) Erfinder: Hermann, Werner, CH-6312 Steinhausen (CH)
(74) Vertreter: Hotz, Klaus, Dipl.-El.-Ing./ETH Patentanwalt

(56) Entgegenhaltungen:
- EP-A- 0 234 811
- EP-A- 0 482 320
- WO-A-86/02261
- WO-A-93/03687
- US-A- 5 019 105

## Beschreibung

Die Erfindung betrifft den Pfannenteil einer Gelenkprothese, insbesondere den Pfannenteil einer Hüftgelenks-Prothese, mit einer zur Verankerung im Knochen bestimmten Mantelhülse und einer in der Mantelhülse mittels Konussitz eingesetzten Gelenkpfanne. Derartige Pfannenteile einer Gelenkprothese sind schon aus der EP-A-0 234 811, WO-A-9 303 687 und US-A-5 019 105 bekannt.

Bei Hüftgelenk-Prothesen wird in der Regel der Pfannenteil in vorgenannter Weise zweiteilig ausgeführt. Dadurch wird die Möglichkeit geschaffen, für die Gelenkpfanne den jeweils geeignet erscheinenden Werkstoff (Keramik, Kunststoff, Metall) zu wählen, vor allem aber auch, den Pfannenteil bei bereits implantierter Mantelhülse auszuwechseln. Der Anlass zur Auswechslung kann sich bereits bei der Erst-Implantation der Hüftgelenk-Prothese ergeben (z.B. Wahl eines anderen Kugelradius), oder aber später bei einer weiteren Operation (z.B. infolge fortgeschrittener Abnützung, Rissen, Unverträglichkeit des Materials der Gelenkpfanne usw.). Mit bisherigen Konstruktionen des zweiteiligen Pfannenteils bereitet allerdings der Ausbau der Gelenkpfanne aus der Mantelhülse meist erhebliche Schwierigkeiten, weil er kaum mit Werkzeugen gefasst werden kann und oft nur die gewaltsame Zertrümmerung übrigbleibt. Jedenfalls sind damit erhebliche Krafteinwirkungen bzw. Schläge auf die Mantelhülse verbunden, wodurch deren Sitz im Hüftknochen gefährdet werden kann.

Ähnliche Überlegungen ergeben sich auch für andere künstliche Gelenke wie zum Beispiel künstliche Schultergelenke.

Ziel der vorliegenden Erfindung ist es, eine geeignete Ausbildung des Pfannenteils vorzuschlagen, um ein Auswechseln der Gelenkpfanne erheblich zu erleichtern und zu beschleunigen. Insbesondere soll beim Ausbau der Gelenkpfanne aus der Mantelhülse die letztere bzw. deren Verankerung im Hüftknochen möglichst wenig belastet werden. Die selbe Problemstellung bildet auch die Basis für die Lösungen der EP-A-0 234 811, WO-A-9 303 687 und US-A-5 019 105.

Diese Aufgabe wird erfindungsgemäss mit der Konstruktion gemäss kennzeichnendem Teil des Patentanspruches 1 gelöst. Die Verwendung einer Zwischenhülse gemäss der Erfindung bedeutet nur geringen Mehraufwand und kaum zusätzlichen Platzbedarf. Hingegen ist es nunmehr möglich, die Gelenkpfanne nur durch Einwirkung einer Abdrückkraft zwischen einem Widerlager am Rand der Zwischenhülse und der Mantelhülse auszubauen, d.h. praktisch ohne Beanspruchung der Verankerung der Mantelhülse im Knochen. Dadurch wird das Auswechseln der Gelenkpfanne stark vereinfacht und beschleunigt, mit entsprechenden Erleichterungen für den Patienten wie für den Arzt. In diesem Zusammenhang ist es auch wichtig, dass die erforderlichen Manipulationen ohne weiteres von vorn (aus der Richtung der Achse der Zwischenhülse) möglich sind, da bei im Knochen eingesetzter Mantelhülse die Zugänglichkeit von der Seite her (radial zur genannten Achse) sehr eingeschränkt ist. - Die abhängigen Patentansprüche beziehen sich auf einzelne konkrete, besondere Ausführungsformen der Erfindung. Im Folgenden werden entsprechende Ausführungsbeispiele in Verbindung mit den Zeichnungen näher beschrieben:
- **Fig. 1**: zeigt die Einzelteile Gelenkpfanne, Zwischenhülse und Mantelhülse je im Längsschnitt gemäss einem ersten Ausführungsbeispiel,
- **Fig. 2**: zeigt die Teile nach Fig. **1** zusammengebaut,
- **Fig. 3,4 und 5**: als vergrösserte Detailausschnitte zeigen in analoger Darstellung jeweils weitere Ausführungsbeispiele; in den **Fig. 2, 3, 4** und **5** sind zusätzlich beim Ausbau zu verwendende Abdrückwerkzeuge dargestellt.

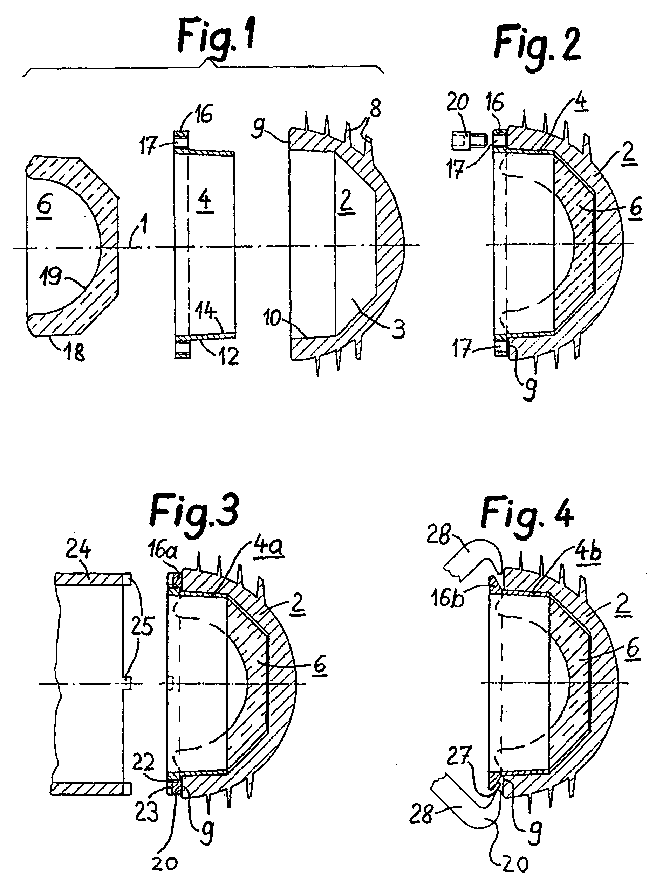

Der Pfannenteil einer Hüftgelenk-Prothese besteht gemäss Ausführungsbeispiel nach Fig. **1** und **2** aus einer Mantelhülse **2**, einer Zwischenhülse **4** und einer Gelenkpfanne **6**. Wie ersichtlich, sind die drei Teile im wesentlichen rotationssymmetrisch bezüglich der Achse **1**. Die Mantelhülse **2** ist zur Verankerung im Knochen (nicht dargestellt) des Patienten bestimmt und zu diesem Zweck an der Aussenseite z.B. mit einem selbstschneidenden Gewinde **8** versehen; es können jedoch durchaus auch andere, an sich bekannte Verankerungsarten in Frage kommen. Zur auswechselbaren Aufnahme der Gelenkpfanne **6** weist die Mantelhülse **2** eine Ausnehmung **3** auf.

Die Gelenkpfanne **6** weist eine die Artikulation des Gelenks ermöglichende, konkave Kugelfläche **19** auf, die in bekannter Weise der Aussenfläche einer zur Gelenkprothese gehörenden Gelenkkugel (nicht dargestellt) angepasst ist. Während die Mantelhülse **2** normalerweise aus Metall gefertigt ist, stehen Gelenkpfannen **6** aus verschiedenen Werkstoffen zur Verfügung, insbesondere aus Keramik, Kunststoff oder Metall-Legierungen.

Um einerseits einen zuverlässigen und genauen Passitz der Gelenkpfanne in der Mantelhülse zu gewährleisten, aber dennoch das einfache Auswechseln der Gelenkpfanne bei bereits implantierter Mantelhülse zu ermöglichen, wird erfindungsgemäss eine konische Zwischenhülse **4** verwendet, welche die beiden Teile **2** und **6** miteinander lösbar verbindet. Die Zwischenhülse **4** bildet mit ihrer konischen Aussenfläche **12** eine selbsthemmende Konusverbindung zu einer entsprechenden Konus-Innenfläche **10** an der Mantelhülse **2**. Ebenso bildet die konische Innenfläche **14** der Zwischenhülse **4** mit einer entsprechenden Konus-Aussenfläche **18** an der Gelenkpfanne **6** eine selbsthemmende Konusverbindung. Die Bedingungen für Selbsthemmung bei Konusverbindungen sind bekannt; vorzugsweise wird ein Konusverhältnis von etwa 1:10 verwendet, und zwar gleich für die Aussen- und Innenflächen **12** bzw. **14** der Zwischenhülse **4**, um zum Beispiel eine dünne Zwischenhülse **4** in Metall ausführen zu können.

Bekanntlich wird bei solchen selbsthemmenden Konusverbindungen bereits mit relativ leichten Schlägen in Axialrichtung ein fester, zuverlässiger Sitz erzielt. Der Zusammenbau des Pfannenteils kann entweder bereits vor der Implantation durch den Hersteller oder den Orthopäden erfolgen, oder auch erst anlässlich der Operation selbst, nachdem die Mantelhülse bereits implantiert ist.

Die Teile sind so dimensioniert, dass im zusammengebauten Zustand (**Fig. 2**) der Sitz ausschliesslich über die genannten Konusflächen erfolgt, d.h. die Gelenkpfanne **6** liegt weder am Grund der Ausnehmung **3** noch an der Stirnseite **9** der Mantelhülse **2** auf. Die konische Zwischenhülse **4** ist an ihrem Rand **16** mit einem Widerlager versehen, das das Ansetzen einer Abdrückvorrichtung **20** erlaubt, um eine Abdrückkraft zwischen Widerlager und Stirnseite **9** der Mantelhülse **2** zu erzeugen. Der Rand **16** kann als durchgehender Ringflansch oder mit einzelnen Flanschabschnitten, die durch Zwischenräume unterbrochen sind, gestaltet sein. Der Rand **16** ist als Widerlager für eine an der Zwischenhülse **4** angreifenden Abdrückvorrichtung **20** ausgebildet; hierfür sind verschiedene Varianten dargestellt, die nachstehend beschrieben werden:

Beim Beispiel nach **Fig. 1, 2** weist der Rand **16** einen Flansch mit einer Anzahl (z.B. 3, 4 oder 6) am Umfang verteilter, durchgehender Gewindebohrungen **17** als Widerlager auf. Zwecks nachträglichem Ausbau einer eingesetzten Gelenkpfanne **6** werden Gewindestifte **20** in die Gewindebohrungen **17** eingeschraubt, bis sie gegen die Stirnseite **9** der Mantelhülse drücken und die Zwischenhülse **4** mit der Gelenkpfanne **6** herausheben.

Die Ausführungsvarianten nach **Fig. 3** und **4** unterscheiden sich vom Beispiel nach **Fig. 1, 2** praktisch nur in der Gestaltung der Zwischenhülse (**4a, 4b**) bzw. von deren Rand (**16a, 16b**).

Beim Beispiel nach **Fig. 3** ist das Widerlager durch ein Feingewinde **22** an der Hülse 4a gebildet, wobei eine Ringmutter **20'**mit pasendem Gegengewinde aufsetzbar ist. Letztere weist eine Anzahl Einkerbungen **23** auf, an denen ein Rohr-Steckschlüssel **24** mit passenden stirnseitigen Nasen **25** angesetzt werden kann. Bei Drehung der Ringmutter **20**'gegenüber dem Gewinde **22** drückt diese gegen die Stirnseite **9** der Mantelhülse und löst so die selbsthemmende Konusverbindung zwischen der letzteren und der Zwischenhülse **4a**. Ein entsprechendes Gegenmoment kann mit einem zweiten Rohrsteckschlüssel und mit einer stirnseitigen Zahnung auf dem Rand der Zwischenhülse erzeugt werden.

Die Zwischenhülse **4b** bei der Ausführungsvariante nach **Fig. 4** weist einen Rand **16b** mit einem radialen Überstand auf, dessen der Mantelhülse bzw. deren Stirnseite **9** zugekehrte Seite **27** angeschrägt ist (es kann sich um einzelne, lokale Anschrägungen handeln, oder um einen durchgehend über den ganzen Umfang angeschrägten Flansch). Dies ermöglicht es, zum Lösen der Zwischenhülse **4b** als Abdrückvorrichtung **20** ein zangenartiges Ausziehwerkzeug **28** in die keilförmige Aussparung zwischen Rand **16b** und Stirnseite **9** einzupressen, wie in **Fig. 4** angedeutet ist.

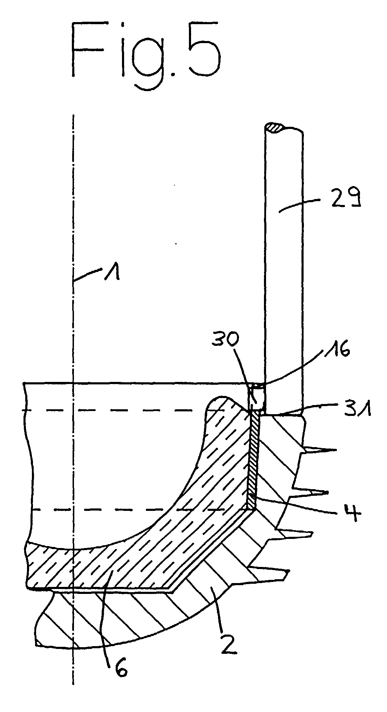

Bei dem Ausführungsbeispiel der **Fig. 5** ist der Rand **16** der Zwischenhülse **4** soweit über die Stirnfläche der Mantelhülse **2** vorstehend, dass eine radiale Querbohrung zugänglich ist, in die ein Zapfen **30** von einem Exzenterhebel **29** einsteckbar ist, um mit einer zum Zapfen **30** exzentrisch verlaufenden Fläche **31** die Zwischenhülse **4** an der Stirnfläche **9** der Mantelhülse **2** abzudrücken.

Ebenso ist es möglich mit einer Spreizvorrichtung im Widerlager auf der Innenseite vom Rand **16** einzufahren und die Spreizvorrichtung zum Abdrücken der Zwischenhülse **4** auf der Stirnfläche **9** der Mantelhülse **2** abzustützen.

Wie aus dem Vorstehenden erkennbar ist, werden beim Lösen der Zwischenhülse mit Hilfe der durch ihren Rand gebildeten Widerlager im wesentlichen nur Kräfte zwischen der Mantelhülse und der Zwischenhülse wirksam, d.h. die Verankerung der Mantelhülse im Knochen wird praktisch nicht belastet. Ausserdem bleibt die Gelenkpfanne unbeschädigt, weil sie zusammen mit der Zwischenhülse herausgehoben wird. Von Bedeutung ist auch die Zugänglichkeit bzw. die Ausführung der nötigen Manipulationen "von vorn" (d.h. in Richtung der Achse **1**), da bei implantiertem Pfannenteil die Zugänglichkeit von der Seite sehr eingeschränkt ist. Im weiteren ist es sinnvoll dem Rand der Zwischenhülse im Bereich der Widerlager genügend Steifigkeit gegen Stülpung zu verleihen.

## Patentansprüche

1. Pfannenteil einer Gelenkprothese, insbesondere einer Hüftgelenk-Prothese, mit einer zur Verankerung im Knochen bestimmten Mantelhülse (**2**) und einer in der Mantelhülse mittels Konussitz eingesetzten Gelenkpfanne (6),
dadurch **gekennzeichnet,**
dass Mantelhülse (**2**) und Gelenkpfanne (**6**) über eine konische Zwischenhülse (**4**) miteinander lösbar verbunden sind, wobei die Zwischenhülse (**4**) einerseits mit ihrer Aussenfläche (**12**) zur Mantelhülse (**2, 10**) und anderseits mit ihrer Innenfläche (**14**) zur Gelenkpfanne (**6, 18**) je eine selbsthemmende Konusverbindung herstellt und ferner einen Rand (**16, 16a, 16b**) aufweist, der als Widerlager ausgebildet ist, um mit einer Abdrückvorrichtung (**20**) eine Abdrückkraft zwischen dem Widerlager und einer Stirnfläche (**9**) der Mantelhülse (**2**) zu erzeugen.

2. Pfannenteil nach Anspruch **1**,
dadurch **gekennzeichnet,**
dass der Rand (**16**) als Flansch mit durchgehenden Gewindebohrungen (**17**) ausgeführt ist, in die Gewindestifte (**20**) einschraubbar sind, welche gegen die Stirnfläche (**9**) der Mantelhülse (**2**) drücken.

3. Pfannenteil nach Anspruch **1**,
dadurch **gekennzeichnet,**
dass der Rand (**16a**) der Zwischenhülse (**4a**) ein Widerlager in Form eines Feingewindes (**22**) aufweist, an welchem eine Ringmutter (**20**') aufsetzbar ist, um bei deren Drehung gegen die Stirnfläche (**9**) der Mantelhülse (**2**) zu drücken.

4. Pfannenteil nach Anspruch **1**,
dadurch **gekennzeichnet,**
dass der Rand (**16b**) einen radialen Überstand aufweist, dessen der Stirnseite (**9**) der Mantelhülse (**2**) zugekehrte Seite (**27**) angeschrägt ist, um von einem Ausziehwerkzeug (**28**) hintergriffen und gegenüber der Mantelhülse (**2**) abgedrückt zu werden.

5. Pfannenteil nach einem der vorangehenden Ansprüche,
dadurch **gekennzeichnet,**
dass der Rand der Zwischenhülse (**4**) als durchgehender Ringflansch ausgeführt ist.

6. Pfannenteil nach einem der Ansprüche **1** bis **4**,
dadurch **gekennzeichnet,**
dass der Rand (**16**) der Zwischenhülse (**4**) einzelne Flanschabschnitte aufweist, die durch Zwischenräume getrennt sind.

7. Pfannenteil nach Anspruch **1**,
dadurch **gekennzeichnet,**
dass der Rand (**16**) der Zwischenhülse (**4**) radiale -ein Widerlager bildende- Einbuchtungen aufweist.

8. Pfannenteil nach einem der Ansprüche **1** bis **7**,
dadurch **gekennzeichnet,**
dass die Zwischenhülse (**4**) aus Metall hergestellt ist.

9. Pfannenteil nach einem der Ansprüche **1** bis **8**, mit einer Abdrückvorrichtung zur Demontage des Pfannenteils, welche
an einem Widerlager am Rand (**16, 16a, 16b**) der Zwischenhülse (**4**) ansetzbar ist, wodurch eine Abdrückkraft gegenüber der Stirmnfläche (**9**) der Mantelhülse (**2**) erzielbar ist.

## Claims

1. Socket of a joint prosthesis, more particularly of a hip-joint prosthesis, having an encasing sleeve (2) intended to provide anchorage in the bone, and a joint cavity (6) which is inserted in a tapering fit in the encasing sleeve,
characterised in that
the encasing sleeve (2) and the joint cavity (6) are removably joined to one another via a conical intermediate sleeve (4), the intermediate sleeve (4) making a respective self-inhibiting tapered connection, on the one hand by its external surface (12) with the encasing sleeve (2, 10), and on the other hand by its internal surface (14) with the joint cavity (6, 18), and also presenting a rim (16, 16a, 16b), constructed in the form of an abutment, in order with a pressure device (20) to generate a squeezing-off force between the abutment and one end surface (9) of the encasing sleeve (2).

2. Socket according to claim 1,
characterised in that
the rim (16) is configured as a flange with threaded through-bores (17) into which threaded pins (20) can be screwed which press against the end surface (9) of the encasing sleeve (2).

3. Socket according to claim 1,
characterised in that
the rim (16a) of the intermediate sleeve (4a) incorporates an abutment in the form of a fine thread (22) onto which a ring nut (20') can be placed in order to press against the end surface (9) of the encasing sleeve (2) whilst said intermediate sleeve is rotated.

4. Socket according to claim 1,
characterised in that
the rim (16b) has a radial overhang of which the side (27) directed towards the end face (9) of the encasing sleeve (2) is angled upwards in order to be engaged from behind by an extracting tool (28) and squeezed off relative to the encasing sleeve (2).

5. Socket according to any of the preceding claims,
characterised in that
the rim of the intermediate sleeve (4) is configured as a continuous ring flange.

6. Socket according to any of claims 1 to 4,
characterised in that
the rim (16) of the intermediate sleeve (4) has individual flange sections which are separated by gaps.

7. Socket according to claim 1,
characterised in that
the rim (16) of the intermediate sleeve (4) has radial indentations which form an abutment.

8. Socket according to any of claims 1 to 7,
characterised in that
the intermediate sleeve (4) is made of metal.

9. Socket according to any of claims 1 to 8, with, for the removal of the socket, a pressure device which is adapted to be placed against an abutment on the rim (16, 16a, 16b) of the intermediate sleeve (4), thereby enabling a squeezing-off force to be obtained relative to the end surface (9) of the encasing sleeve (2).

## Revendications

1. Cupule d'une prothèse d'articulation, notamment d'une prothèse d'articulation de la hanche, comportant un manchon formant enveloppe (2) destiné à être ancré dans l'os et une cuvette articulaire (6) insérée dans le manchon formant enveloppe par ajustement conique,
caractérisée
en ce que le manchon formant enveloppe (2) et la cuvette articulaire (6) sont reliés entre eux de manière non permanente par un manchon intermédiaire conique (4), le manchon intermédiaire (4) réalisant une liaison conique autobloquante, d'une part avec le manchon formant enveloppe (2, 10) par sa surface extérieure (12), et d'autre part avec la cuvette articulaire (6, 18) par sa surface intérieure (14), et présentant en outre un bord (16, 16a, 16b) qui est réalisé sous la forme d'une butée afin de produire, au moyen d'un dispositif de séparation (20), une force de séparation entre la butée et une face frontale (9) du manchon formant enveloppe (2).

2. Cupule selon la revendication 1,
caractérisée
en ce que le bord (16) est réalisé sous la forme d'une bride avec trous taraudés (17) débouchants, dans lesquels peuvent être vissées des tiges filetées (20) qui pressent contre la face frontale (9) du manchon formant enveloppe (2).

3. Cupule selon la revendication 1,
caractérisée
en ce que le bord (16a) du manchon intermédiaire (4a) présente une butée sous la forme d'un filetage fin (20, 22) sur lequel peut être placé un écrou annulaire (20'), afin de presser, lors de sa rotation, contre la face frontale (6) du manchon formant enveloppe (2).

4. Cupule selon la revendication 1,
caractérisée
en ce que le bord (16b) présente un porte-à-faux radial dont le côté (27), tourné vers la face frontale (9) du manchon formant enveloppe (2), est chanfreiné, afin que ce côté puisse être saisi de l'arrière par un outil de vissage (28) et qu'il puisse être pressé par rapport au manchon formant enveloppe (2).

5. Cupule selon l'une des revendications précédentes,
caractérisée
en ce que le bord du manchon intermédiaire (4) est réalisé sous la forme d'une bride annulaire continue.

6. Cupule selon l'une des revendications 1 à 4,
caractérisée
en ce que le bord (16) du manchon intermédiaire (4) présente des portions individuelles de bride qui sont séparées par des interstices.

7. Cupule selon la revendication 1,
caractérisée
en ce que le bord (16) du manchon intermédiaire (4) présente des dentelures radiales qui forment une butée.

8. Cupule selon l'une des revendications 1 à 7,
caractérisée
en ce que le manchon intermédiaire (4) est en métal.

9. Cupule selon l'une des revendications 1 à 8 avec un dispositif de séparation pour le démontage de la cupule, qui peut être placé contre une butée sur le bord (16, 16a, 16b) du manchon intermédiaire (4), ce qui permet d'obtenir une force de séparation par rapport à la face frontale (9) du manchon formant enveloppe (2).
